# EUROPEAN PATENT SPECIFICATION

(11) **EP 3 397 707 B1**
(45) Date of publication and mention of the grant of the patent: **20.11.2019**
(21) Application number: 16831851.7
(22) Date of filing: 16.12.2016
(51) Int. Cl.: C09D 195/00, C08L 95/00

(54) **A SELF-HEALING MODIFIED BITUMEN COMPOSITION FOR USE IN ASPHALT PRODUCTION, AND METHOD OF PRODUCING SAME**
SELBSTREGENERIERENDE VERÄNDERTE BITUMEN ZUSAMMENSETZUNG ZUR VERWENDUNG IN DER ASPHALTHERSTELLUNG UND VERFAHREN ZU DESSEN HERSTELLUNG
COMPOSITION DE BITUME AUTO-RÉPARATRICE MODIFIÉ POUR LA PRODUCTION D'ASPHALTE ET MÉTHODE DE PREPARATION DE CELLE-CI

(30) Priority: 30.12.2015 TR 201517516
(43) Date of publication of application: 07.11.2018
(73) Proprietor: Turkiye Petrol Rafinerileri Anonim Sirketi Tupras, 41780 Korfez/Kocaeli (TR)
(72) Inventor: CANIAZ, Ramazan Oguz, 41790 Korfez/Kocaeli (TR); CETINTAS, Refika, 41790 Korfez/Kocaeli (TR); ARCA, Serhat, 41790 Korfez/Kocaeli (TR); BASKENT, Emel, 41790 Korfez/Kocaeli (TR); KOCAMAN, Elif, 41790 Korfez/Kocaeli (TR); KOSTERELI, Ziya, 41790 Korfez/Kocaeli (TR); YASAR, Muzaffer, 34320 Istanbul (TR); GURDAL, Savas, Esenyurt/Istanbul (TR); KOMURCU, Hasan, 34420 Beyoglu/Istanbul (TR); ABDULLA, Ali, Bahcelievler/Istanbul (TR); MEDINA CABELLO, Francisco, 43007 Tarragona (ES)
(74) Representative: Cayli, Hülya
(86) International application number: PCT/TR2016/050502
(87) International publication number: WO 2017/116354

(56) References cited:
- US-A- 3 014 809
- US-A- 6 045 608

## Description

### Technical Field of the Invention

The present invention relates to a modified bitumen composition which is used as a binding raw material in asphalt-coated roads and which can heal itself with the ionic liquid as an additive comprised therein, and a method of producing same.

### Background Art

Bitumen, which is a liquid with a high viscosity and consisted of molecules containing heavy molecular weight hydrocarbon and heteroatom, is produced as a bottom product of vacuum distillation units of refineries. By mixing bitumen having binding properties with aggregate, asphalt mixtures are prepared, which are then used to pave roads. In asphalt mixtures, about 5% bitumen - 95% aggregate is present. Although the proportion of bitumen in asphalt mixtures appears to be low, the bitumen is the most important factor in determining performance of an asphalt coating due to its binding properties.

Although bitumen is a durable material, various problems such as thermal cracks, fatigue-induced cracks and rutting problems are experienced in asphalt-coated roads, based on the usage area and climate conditions. Due to the cracks and rutting problems on the asphalt, it is necessary to periodically make maintenance of asphalts and to renew them, where necessary. This increases road maintenance and repair costs.

In prior art, in order to prevent cracks and rutting problems on asphalts, bitumen is modified with various additives. The most commonly used additive among them is SBS polymer. WO2009013339A discloses a polymer-modified bitumen composition designed to avoid incompatibility problem of polymer and bitumen. The additive SBS increases strength of bitumen, without however having a remarkable effect on the self-healing property of bitumen. SBS improves high-temperature performance of bitumen, but cannot create a positive effect on its low-temperature performance. Given that thermal cracks mainly occur at low temperatures, it is apparent that polymer will not be helpful in low temperature climate conditions. Additionally, SBS additive does not have a significant positive effect on binding of bitumen and aggregate. In this respect, SBS additive does not provide improved resistance against adhesive crack formation that occurs between bitumen and aggregate molecules in asphalt coatings.

In addition to the additives used in bitumen modification to increase strength of bitumen, additives intended to improve self-healing properties of bitumen are also used. If the teachings of patents for improving "self-healing properties" of bitumen/asphalt or various construction materials, several products and methods will be seen, as detailed below, wherein various additives from bacteria to fiber materials are used in the mixture.

EP2247551 B1 in the prior art discloses incorporation of various living organisms into cement-based materials in order to improve their self-healing properties. Here, calcite precipitating bacteria is added so that self-healing is achieved by formation of metabolic or enzymatic biominerals. However, self-healing phenomenon achieved using bacteria as specified in EP2247551 B1 can take place at high pH values at which bacteria maintain their viability.

In CN102786258 (A), viscosity of asphalt binder is increased by heating conductive fiber materials added into the asphalt mixture and binding properties are improved, so that cracks that have occurred in asphalt coating can be repaired. However, self-healing phenomenon achieved therein by heating of fiber materials requires external heating.

### Brief Description of the Invention

With the present invention, there is provided a self-healing modified bitumen composition that is used as a binding raw material in asphalt-coated roads, and a method of producing same. Said modified bitumen composition comprises bitumen with a penetration value of 20 to 120 and as an additive, at least one ionic liquid of 0.1 to 20 % by weight wherein said ionic liquid is an ionic liquid from the imidazolium group. Said production method comprises the steps of heating bitumen with a penetration value of 20 to 120 to a predetermined temperature so as to fluidize it; mixing fluidized bitumen by means of a mixer for a first predetermined period of time; adding a portion of ionic liquid from the imidazolium group as an additive into bitumen; mixing additive-incorporated bitumen by means of the said mixer for a second predetermined period of time; repeating the steps of adding additive and mixing additive-incorporated bitumen until all of the additive is added to bitumen; after all of the additive is added, mixing the resulting mixture by means of the said mixer for a third predetermined period of time so as to obtain modified bitumen composition.

In the modified bitumen composition according to the present invention, self-healing properties at both low-temperatures and high-temperatures is imparted to bitumen by using an ionic liquid as an additive, and also binding of modified bitumen composition to aggregate is increased. Said improvements are achieved by means of bonds formed by polar groups of ionic liquids as well as dissolution in bitumen because of alkyl chains.

Several additives can be used in order to increase self-healing/asphalt performance of bitumen. Various additives such as fiber, bacteria etc. are used in order to increase self-healing properties, with polymers being the most common additives to improve performance of bitumen/asphalt. Nowhere in the literature is disclosed a similar product content and production method according to this invention, wherein ionic liquids are used in bitumen or asphalt modification alone and/or binary and/or in a multiple manner in order to improve self-healing/asphalt performance.

### Object of the Invention

An object of the present invention is to provide a self-healing modified bitumen composition used as a binding raw material in asphalt-coated roads, and a method of producing the modified bitumen composition .

Another object of the present invention is to provide a modified bitumen composition that improves nano-, micro- and meso-scale cracks on the asphalt without reaching to macro scales, and a method of producing same.

The most important advantage of the present invention over similar ones is that self-healing property is not restricted depending on different external factors. In accordance with the invention, self-healing property is achieved by means of bonds formed by polar groups of ionic liquids as well as dissolution in bitumen because of alkyl tails, which distinguishes the present invention from the other inventions.

Another object of the present invention is to provide a modified bitumen composition having a high self-healing performance both at high temperatures and low temperatures by means of ionic and/or weak bonds formed by polar groups of ionic liquids with polar groups in aggregate and bitumen and by means of dissolution in bitumen because of alkyl chains, and a method of producing said modified bitumen composition.

Another object of the present invention is to provide a modified bitumen composition with improved binding to aggregate, and a method of producing same.

Still another object of the present invention is to provide a modified bitumen composition with improved asphalt performance and increased resistance against fatigue, and a method of producing same.

### Description of the Drawings

Illustrative embodiments of the modified bitumen composition according to the present invention are illustrated in the enclosed drawings, in which:
Figure 1 is a test result of Zeta potential of an embodiment of the modified bitumen composition according to the present invention.
Figure 2 is a test result of Zeta potential of another embodiment of the modified bitumen composition according to the present invention.

### Description of the Invention

Asphalt is achieved by mixing bitumen which is a bottom product of vacuum distillation units of petroleum refineries with aggregate. Bitumen allows aggregate particles to bind each other. Cracks or rutting may occur in bitumen-used asphalts due to environmental effects (for example temperature or pressure). This increases road maintenance and repair costs. Therefore, with the present invention, there is provided a modified bitumen composition that heals itself so as to minimize such problems as cracks and rutting on asphalt, and a method of producing said modified bitumen composition.

The modified bitumen composition according to the present invention comprises bitumen with a penetration value of 20 to 120 (preferably 40 to 80) and as an additive, at least one ionic liquid of 0.1 to 20 % by weight (preferably 0.1 to 5%) wherein said ionic liquid is an ionic liquid from the imidazolium group. Due to incorporation of an ionic liquid into bitumen as an additive, self-healing and aggregate binding properties of bitumen are improved by means of the bonds formed by polar groups of ionic liquids and also the dissolution in bitumen because of alkyl tails. By using different types and amounts of ionic liquids in the formulations, modified bitumen compositions can be produced which are suitable for different climate and traffic conditions and which heal nano-, micro- and meso-scale cracks on asphalt without reaching to macro scales. Thus, asphalt coatings produced by mixing the inventive modified bitumen composition with at least one aggregate are also included within the scope of the invention.

The production method of modified bitumen composition according to the invention comprises the steps of heating bitumen with a penetration value of 20 to 120 (preferably 40 to 80) to a predetermined temperature (e.g. 90-120°C, more specifically 100°C) so as to fluidize it; mixing fluidized bitumen by means of a mixer for a first predetermined period of time (e.g. 30 minutes); adding a portion of ionic liquid from the imidazolium group as an additive (e.g. a portion of 1%) into bitumen; mixing additive-incorporated bitumen by means of the said mixer for a second predetermined period of time (e.g. 3 minutes); repeating the steps of adding additive and mixing additive-incorporated bitumen until all of the additive is added to the bitumen; after all of the additive is added, mixing the resulting mixture by means of the said mixer for a third predetermined period of time (e.g. 30 minutes) so as to obtain modified bitumen composition. At the step of mixing fluidized bitumen, the mixing rate is preferably 250 rpm. Similarly, at the step of mixing additive-incorporated bitumen and/or at the step of mixing the resulting mixture after all of the additive is added, the mixing rate is 250 rpm.

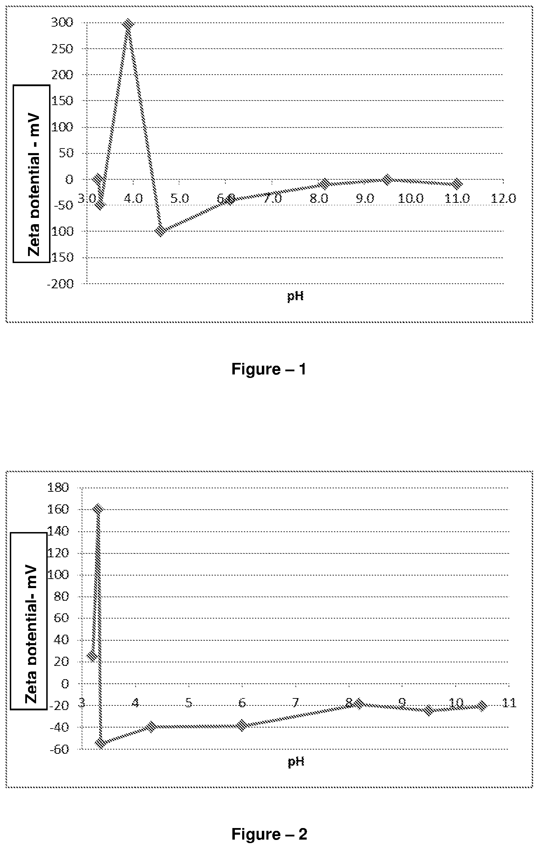

In an illustrative embodiment of the invention, 1 butyl-3-methyl imidazolium chloride is used as additive, the percentage of the additive is 0.1 to 5 % by weight, and bitumen with a penetration of 40 to 80 is used as bitumen. The improvement values of the modified bitumen composition obtained in this embodiment as compared to additive-free bitumen are given in Table 1. For comparison of said improvement values, a standard MSCR (Multiple Stress Creep Recovery) test is modified in the present case, so that the effect of the relaxation (recovery) time and test temperature on the self-healing performance of the modified bitumen composition and additive-free bitumen is determined. In addition, in order to determine self-healing properties of the modified bitumen composition at low temperature, a new method is developed in Fraass Breaking Point Apparatus, then the results of the modified bitumen composition and additive-free bitumen obtained using this method are compared. In the said method, samples are exposed to bending in Fraass Breaking Point apparatus for a certain period of time at low temperature and at a speed avoiding any breakage of samples, followed by being exposed to relaxation (recovery) for different periods of time, at the end of which they are bended at a higher bending speed until a breakage occurs on the sample. Thereafter, the time periods that have elapsed until breakage is seen on the sample are compared with the aim of determining low-temperature performance of the modified bitumen compositions. In addition to these tests, stripping strength test is also performed in order to determine interaction of the modified bitumen composition with aggregate. Furthermore, bitumen-fine aggregate/filler mixtures are prepared in order to measure electrostatic interactions between the modified bitumen composition and aggregate/filler material. Zeta potential measurements are performed on the mixtures prepared, which are based on applying an electric field to the dispersion and determination of attraction rate of particles in the dispersion against reverse polarity electrodes, and the relevant test results are provided in Table 1 and Figure 1.

**Table - 1**

| **Experiment** | **Improvement over Additive-free Bitumen (%)** |
|---|---|
| Modified MSCR (at 64 °C) | 17.5 |
| Modified MSCR (at 70 °C) | 32.4 |
| Method Designed in Fraass Breaking Point Apparatus | 210 |
| Stripping Strength Test | 7.8 |

From the results in Table 1, it is clear that self-healing property of ionic liquid added bitumen composition is much better than the additive-free bitumen. Given that ionic liquids are good solvents for various substances such as hydrocarbons etc., improvements obtained in the modified MSCR test intended to measure the effect of relaxation (recovery) period at high temperatures on self-healing property may be associated with closing of micro-scale cracks due to local mobilization imparted to bitumen by the ionic liquids. The improvements obtained in the test designed in Fraass Breaking Point apparatus as well as the Stripping Strength tests can be explained in that polar portions of ionic liquids strengthen attraction between bitumen-aggregate molecules / allow formation of bonds. With the formulation specified in this embodiment, self-healing property in bitumen phase both at low temperatures and high temperatures is able to be improved. As part of the stability studies conducted to determine storage and transportation compatibility of the developed products, in order to determine whether the modified bitumen compositions maintain their homogeneity when stored prior to asphalt mixture (during storage in the refinery field and/or transportation to asphalt plants), the modified bitumen compositions are stored for different period of times (1 day, 1 week, 1 month) and then viscosity of the bitumens is measured and viscosity change of the bitumens stored for different periods of time is observed. The relevant observation results are provided in Table 2. The results show that if the prepared modified bitumen compositions are stored for 1 week or up to 1 month, a viscosity change of 2.5% is experienced, thus the products do not lose their homogeneity. In this respect, the inventive product formulations will not loss their homogeneity during storage, transportation etc. until they are supplied to the end users, and will ensure high performance after application.

**Table - 2**

| **Sample** | **Viscosity (Cp)** |
|---|---|
| Sample on day 1 | 488 |
| Sample on day 7 | 475 |
| Sample on day 30 | 475 |

In another illustrative embodiment of the invention, 1-hexadecyl-3-methyl imidazolium bis trifluoromethylsulfonyl is used as additive, the percentage of the additive is 0.1 to 5 % by weight, and bitumen with a penetration of 40 to 80 is used as bitumen. The improvement values of the modified bitumen composition obtained in this embodiment as compared to additive-free bitumen are given in Table 3. Furthermore, with the product prepared to determine asphalt performance of the developed formulation, asphalt samples are prepared using aggregate whose sieve analysis results are specified in Table 4, and fatigue and stiffness tests are performed on the prepared samples according to standards EN 12697-24E and EN 12697-26C. Bitumen-fine aggregate/filler mixtures are prepared in order to measure electrostatic interactions between the modified bitumen composition and aggregate/filler material, Zeta potential measurements are made on the prepared mixtures and the relevant test results are provided in Table 3, Table 5 and Figure 2.

**Table - 3**

| **Experiment** | **Improvement over Additive-free Bitumen (%)** |
|---|---|
| Stripping Strength Test | 15 |
| Method Designed in Fraass Breaking Point Apparatus | 230 |

**Table 4**

| **USAGE RATE** | | **10%** | **47%** | **43%** | **MIXTURE GRADATION** | **2013 HIGHWAY TECHNICAL SPECIFICATIONS WEARING TYPE-1 LAYER LIMITS** | | | |
|---|---|---|---|---|---|---|---|---|---|
| **SIEVE APERTURE (Inch) (mm)** | | **12-19 mm basalt** | **5-12 mm basalt** | **0-5 mm limest one** | | **SPECIFICATIONS** | | **BUSINESS MIXTURE FORMULATION** | |
| ¾" | 19,0 | 100.0 | | | 100.0 | 100 | | 100 | |
| ½" | 12,5 | 19.4 | 98.9 | | 91.4 | 88 | 100 | 88 | 95 |
| 3/8" | 9,5 | 1.1 | 77.6 | 100.0 | 79.6 | 72 | 90 | 76 | 84 |
| No 4 | 4,75 | 0.8 | 15.1 | 96.8 | 48.8 | 42 | 52 | 45 | 52 |
| No 10 | 2,00 | 0.8 | 2.0 | 59.9 | 26.8 | 25 | 35 | 25 | 30 |
| No 40 | 0,425 | 0.7 | 1.6 | 27.0 | 12.4 | 10 | 20 | 10 | 15 |
| No 80 | 0,180 | 0.6 | 1.2 | 19.6 | 9.1 | 7 | 14 | 7 | 12 |
| No 200 | 0,075 | 0.6 | 1.0 | 12.0 | 5.7 | 3 | 8 | 4 | 8 |

**Table 5**

| **Experiment** | **Improvement over Additive-free Bitumen (%)** |
|---|---|
| Fatigue (EN 12697-24E) | 30 |
| Stiffness (EN 12697-26C) | 15 |

With the formulation in this embodiment, improvements in self-healing property in bitumen phase at low temperatures, resistance against stripping that measures binding of bitumen to aggregate under the effect of water and traffic, and asphalt performance are achieved as compared to additive-free bitumen. Improvement in stripping strength test is confirmed in a sense by the Zeta potential measurements. In view of the improvements made not only in low- temperature performance in bitumen phase but also in resistance against stripping and in asphalt performance, it is determined that this formulation is suitable for use both in low temperature and rainy climate regions and high-traffic load regions. As part of the stability studies conducted to determine storage and transportation compatibility of the developed products, in order to determine whether the modified bitumen compositions maintain their homogeneity when stored prior to asphalt mixture (during storage in the refinery field and/or transportation to asphalt plants), the modified bitumen compositions are stored for different period of times (1 day, 1 week,) and then viscosity of the bitumens is measured and viscosity change of the bitumens stored for different periods of time is observed. The relevant observation results are provided in Table 6. The results show that viscosity change at the end of the 7^{th} day is 2.8%, which means that the prepared modified bitumen compositions will not loss their homogeneity during storage and transportation. In this respect, the inventive product formulations will not loss their homogeneity during storage, transportation etc. until they are supplied to the end users, and will ensure high performance after application.

**Table - 6**

| **Sample** | **Viscosity (Cp)** |
|---|---|
| Sample on day 1 | 438 |
| Sample on day 7 | 425 |

In another illustrative embodiment of the invention, 0.1 to 5% by weight of 1 butyl-3-methyl imidazolium chloride and 0.1 to 5% by weight of 1-hexadecyl-3-methyl imidazolium bis trifluoromethylsulfonyl are used as additive, and bitumen with a penetration of 40 to 80 is used as bitumen. The improvement values of the modified bitumen composition obtained in this embodiment as compared to additive-free bitumen are given in Table 7.

**Table - 7**

| **Experiment** | **Improvement over Additive-free Bitumen (%)** |
|---|---|
| Stripping Strength Test | 30.8 |
| Modified MSCR (at 70 °C) | 17.3 |
| Fatigue (EN 12697-24E) | 122 |

In this embodiment wherein two different ionic liquids are together added into bitumen, it is found that due to dual interactions, more favorable results are obtained as compared to the conditions where ionic liquids are added alone. In case two different ionic liquids are together added into bitumen, it is determined that favorable features imparted to bitumen by the both ionic liquids are maintained and significant improvements are achieved with different features from the situation where ionic liquids are added alone into bitumen. When the results are examined, it is determined that the formulation in this example is suitable for use at hot and rainy climate conditions and high-traffic load regions.

With the present invention, there is provided a modified bitumen composition that heals nano-, micro and meso-scale cracks on asphalt without reaching to macro-scales. Due to the use of an ionic liquid as an additive according to the present invention, self-healing and aggregate binding properties of bitumen are improved by means of the bonds formed by polar groups of ionic liquids and by means of the dissolution in bitumen because of alkyl tails. The modified bitumen composition according to the present invention can also be used in roof coatings.

## Claims

1. A self-healing modified bitumen composition comprising bitumen with a penetration value of 20 to 120 and as an additive, at least one ionic liquid of 0.1 to 20 % by weight **characterized in that** said ionic liquid is an ionic liquid from the imidazolium group.

2. A modified bitumen composition according to claim 1, **characterized in that** the penetration value of bitumen is between 40 and 80.

3. A modified bitumen composition according to claim 1, **characterized in that** the percentage by weight of the additive is 0.1 to 5%.

4. A modified bitumen composition according to claim 1, **characterized in that** the ionic liquid comprises 1 butyl-3-methyl imidazolium chloride.

5. A modified bitumen composition according to claim 1, **characterized in that** the ionic liquid comprises 1-hexadecyl-3-methyl imidazolium bis trifluoromethylsulfonyl.

6. A modified bitumen composition according to claim 1, **characterized in that** the ionic liquid comprises 1 butyl-3-methyl imidazolium chloride and 1-hexadecyl-3-methyl imidazolium bis trifluoromethylsulfonyl.

7. A method of producing a modified bitumen composition according to claims 1-6, **characterized by** comprising the steps of:
- heating bitumen with a penetration value of 20 to 120 to a predetermined temperature so as to fluidize it;
- mixing fluidized bitumen by means of a mixer for a first predetermined period of time;
- adding a portion of ionic liquid from the imidazolium group as an additive into bitumen;
- mixing additive-incorporated bitumen by means of the said mixer for a second predetermined period of time;
- repeating the steps of adding additive and mixing additive-incorporated bitumen until all of the additive is added to bitumen;
- after all of the additive is added, mixing the resultind mixture by means of the said mixer for a third predetermined period of time so as to obtain modified bitumen composition.

8. A method of production according to claim 7, **characterized in that** said predetermined temperature is in the range of 90-120°C.

9. A method of production according to claim 7, **characterized in that** said predetermined temperature is 100°C.

10. A method of production according to claim 7, **characterized in that** said first predetermined time is 30 minutes.

11. A method of production according to claim 7, **characterized in that** said second predetermined time is 3 minutes.

12. A method of production according to claim 7, **characterized in that** said third predetermined time is 30 minutes.

13. A method of production according to claim 7, **characterized in that** at the step of mixing fluidized bitumen and at the step of mixing additive-incorporated bitumen, the mixing rate is 250 rpm.

14. A method of production according to claim 7, **characterized in that** at the step of mixing the resulting mixture after all of the additive is added, the mixing rate is 250 rpm.

15. Asphalt coating produced by mixing the modified bitumen composition produced according to claims 1 to 14 with at least one aggregate.

## Patentansprüche

1. Selbstregenerierende, modifizierte Bitumen-Zusammenset-zung, die Bitumen mit einem Penetrationswert von 20 bis 120 und als ein Additiv mindestens eine ionische Flüssigkeit in 0,1 bis 20 Gew.-% umfasst, **dadurch gekennzeichnet, dass** die ionische Flüssigkeit eine ionische Flüssigkeit aus der Imidazolium-Gruppe ist.

2. Modifizierte Bitumen-Zusammensetzung nach Anspruch 1, **dadurch gekennzeichnet, dass** der Penetrationswert des Bitumens zwischen 40 und 80 liegt.

3. Modifizierte Bitumen-Zusammensetzung nach Anspruch 1, **dadurch gekennzeichnet, dass** der Gewichtsprozentsatz des Additivs 0,1 bis 5% beträgt.

4. Modifizierte Bitumen-Zusammensetzung nach Anspruch 1, **dadurch gekennzeichnet, dass** die ionische Flüssigkeit 1-Butyl-3-methyl-imidazoliumchlorid umfasst.

5. Modifizierte Bitumen-Zusammensetzung nach Anspruch 1, **dadurch gekennzeichnet, dass** die ionische Flüssigkeit 1-Hexadecyl-3-methyl-imidazolium-bis-trifluormethylsulfonyl umfasst.

6. Modifizierte Bitumen-Zusammensetzung nach Anspruch 1, **dadurch gekennzeichnet, dass** die ionische Flüssigkeit 1-Butyl-3-methyl-imidazoliumchlorid und 1-Hexadecyl-3-methyl-imidazolium-bis-trifluormethylsulfonyl umfasst.

7. Verfahren zur Herstellung einer modifizierten Bitumen-Zusammensetzung gemäß einem der Ansprüche 1 bis 6, **dadurch gekennzeichnet, dass** es die Schritte:
- Erhitzen von Bitumen mit einem Penetrationswert von 20 bis 120 auf eine vorbestimmte Temperatur, um ihn zu verflüssigen,
- Mischen des verflüssigten Bitumens mittels eines Mischers über einen vorbestimmten Zeitraum,
- Zugeben einer Portion ionischer Flüssigkeit aus der Imidazolium-Gruppe als ein Additiv in den Bitumen,
- Mischen des Bitumens mit dem eingeführten Additiv mittels des Mischers über einen zweiten vorbestimmten Zeitraum,
- Wiederholen der Schritte des Zugebens von Additiv und des Mischen des Bitumens mit dem eingeführten Additiv, bis Alles von dem Additiv dem Bitumen zugegeben worden ist,
- nachdem Alles von dem Additiv zugegeben worden ist, Mischen der resultierenden Mischung mittels des Mischers über einen dritten vorbestimmten Zeitraum umfasst, um so eine modifizierte Bitumen-Zusammensetzung zu erhalten.

8. Verfahren zur Herstellung nach Anspruch 7, **dadurch gekennzeichnet, dass** die vorbestimmte Temperatur im Bereich von 90-120°C liegt.

9. Verfahren zur Herstellung nach Anspruch 7, **dadurch gekennzeichnet, dass** die vorbestimmte Temperatur 100°C beträgt.

10. Verfahren zur Herstellung nach Anspruch 7, **dadurch gekennzeichnet, dass** der erste vorbestimmte Zeitraum 30 Minuten beträgt.

11. Verfahren zur Herstellung nach Anspruch 7, **dadurch gekennzeichnet, dass** der zweite vorbestimmte Zeitraum 3 Minuten beträgt.

12. Verfahren zur Herstellung nach Anspruch 7, **dadurch gekennzeichnet, dass** der dritte vorbestimmte Zeitraum 30 Minuten beträgt.

13. Verfahren zur Herstellung nach Anspruch 7, **dadurch gekennzeichnet, dass** bei dem Schritt des Mischens des verflüssigten Bitumens und bei dem Schritt des Mischens des Bitumens mit dem eingeführten Additiv die Mischrate 250 upm beträgt.

14. Verfahren zur Herstellung nach Anspruch 7, **dadurch gekennzeichnet, dass** bei dem Schritt des Mischens der resultierenden Mischung, nachdem Alles von dem Additiv zugegeben worden ist, die Mischrate 250 upm beträgt.

15. Asphalt-Beschichtung hergestellt durch Mischen der modifizierten Bitumen-Zusammensetzung, die gemäß den Ansprüchen 1 bis 14 hergestellt worden ist, mit mindestens einem Zuschlagstoff.

## Revendications

1. Composition de bitume modifié autorégénérante comprenant un bitume présentant une valeur de pénétration de 20 à 120 et en tant qu'additif, au moins un liquide ionique de 0,1 à 20 % en poids, **caractérisée en ce que** ledit liquide ionique est un liquide ionique provenant du groupe imidazolium.

2. Composition de bitume modifié selon la revendication 1, **caractérisée en ce que** la valeur de pénétration du bitume est située entre 40 et 80.

3. Composition de bitume modifié selon la revendication 1, **caractérisée en ce que** le pourcentage en poids de l'additif est de 0,1 à 5 %.

4. Composition de bitume modifié selon la revendication 1, **caractérisée en ce que** le liquide ionique comprend du chlorure de 1-butyl-3-méthylimidazolium.

5. Composition de bitume modifié selon la revendication 1, **caractérisée en ce que** le liquide ionique comprend du 1-hexadécyl-3-méthylimidazolium bis trifluorométhylsulfonyle.

6. Composition de bitume modifié selon la revendication 1, **caractérisée en ce que** le liquide ionique comprend du chlorure de 1-butyl-3-méthylimidazolium et du 1-hexadécyl-3-méthylimidazolium bis trifluorométhylsulfonyle.

7. Procédé de production d'une composition de bitume modifié selon les revendications 1 à 6, **caractérisé en ce qu'**il comprend les étapes suivantes :
- le chauffage d'un bitume ayant une valeur de pénétration de 20 à 120 jusqu'à une température prédéterminée afin de le fluidiser ;
- le mélange du bitume fluidisé au moyen d'un mélangeur pendant une première période de temps prédéterminée ;
- l'ajout d'une partie d'un liquide ionique provenant du groupe imidazolium en tant qu'additif au bitume ;
- le mélange du bitume contenant un additif au moyen dudit mélangeur pendant une deuxième période de temps prédéterminée ;
- la répétition des étapes d'ajout d'additif et de mélange du bitume contenant un additif jusqu'à ce que tout l'additif soit ajouté au bitume ;
- après l'ajout de la totalité de l'additif, le mélange du mélange résultant au moyen dudit mélangeur pendant une troisième période de temps prédéterminée afin d'obtenir une composition de bitume modifié.

8. Procédé de production selon la revendication 7, **caractérisé en ce que** ladite température prédéterminée est située dans la plage allant de 90 à 120 °C.

9. Procédé de production selon la revendication 7, **caractérisé en ce que** ladite température prédéterminée est de 100 °C.

10. Procédé de production selon la revendication 7, **caractérisé en ce que** ledit premier temps prédéterminé est de 30 minutes.

11. Procédé de production selon la revendication 7, **caractérisé en ce que** ledit deuxième temps prédéterminé est de 3 minutes.

12. Procédé de production selon la revendication 7, **caractérisé en ce que** ledit troisième temps prédéterminé est de 30 minutes.

13. Procédé de production selon la revendication 7, **caractérisé en ce que** dans l'étape de mélange de bitume fluidisé et dans l'étape de mélange de bitume contenant un additif, la vitesse de mélange est de 250 tours/minutes.

14. Procédé de production selon la revendication 7, **caractérisé en ce que** dans l'étape de mélange du mélange résultant après l'ajout de la totalité de l'additif, la vitesse de mélange est de 250 tours/minutes.

15. Revêtement d'asphalte produit par le mélange de la composition de bitume modifié produite selon les revendications 1 à 14 avec au moins un agrégat.
